# EUROPÄISCHE PATENTSCHRIFT

(11) **EP 0 832 569 B1**
(45) Veröffentlichungstag und Bekanntmachung des Hinweises auf die Patenterteilung: **26.11.2003**
(21) Anmeldenummer: 97115588.2
(22) Anmeldetag: 09.09.1997
(51) Int. Cl.: A23L 1/275

(54) **Herstellung von pulverförmigen, kaltwasserdispergierbaren Carotinoid-Zubereitungen und die Verwendung der neuen Carotinoid-Zubereitnungen**
Preparing powdery carotenoide compounds dispersible in cold water and their use
Préparation de compositions de caroténoide poudreuses, dispersables à froid dans l'eau et leur usage

(30) Priorität: 13.09.1996 DE 19637517
(43) Veröffentlichungstag der Anmeldung: 01.04.1998
(73) Patentinhaber: BASF AKTIENGESELLSCHAFT, 67056 Ludwigshafen (DE)
(72) Erfinder: Auweter, Helmut, Dr., 67117 Limburghof (DE); Bohn, Heribert, 67319 Wattenheim (DE); Haberkorn, Herbert, Dr., 67269 Grünstadt (DE); Horn, Dieter, Dr., 69120 Heidelberg (DE); Lüddecke, Erik, Dr., 67112 Mutterstadt (DE); Rauschenberger, Volker, Dr., 67069 Ludwigshafen (DE)

(56) Entgegenhaltungen:
- DE-A- 2 004 312
- DE-A- 2 440 747
- DE-A- 3 119 383
- GB-A- 918 399
- US-A- 4 726 955
- US-A- 5 364 563

## Beschreibung

Die Erfindung betrifft ein Verfahren zur Herstellung von pulverförmigen, kaltwasserdispergierbaren Carotinoid-Zubereitungen dadurch gekennzeichnet, daß man
a) eine molekulardisperse Lösung eines Carotinoids gegebenenfalls zusammen mit einem Emulgator und/oder einem eßbaren Öl in einem flüchtigen, mit Wasser mischbaren, organischen Lösungsmittel bei höherer Temperatur herstellt und mit einer wäßrigen Lösung eines Schutzkolloids versetzt, wobei die hydrophile Lösungsmittelkomponente in die wäßrige Phase überführt wird und die hydrophobe Phase des Carotinoids als nanodisperse Phase entsteht,
b) das so erhaltene Hydrosol bei einer Temperatur zwischen 40°C bis 90°C für eine Zeitdauer von 5 bis 24 Stunden tempert und
c) das getemperte Hydrosol von dem Lösungsmittel und dem Wasser befreit und in ein wasserdispergierbares Trockenpulver überführt.

Ferner betrifft die Erfindung die so erhältlichen neuen Carotinoid-haltigen, kaltwasserdispergierbaren Trockenpulver, die je nach Herstellungsvariante unterschiedliche Farbwirkungen aufweisen.

Carotinoide bilden eine Gruppe von Farbpigmenten mit gelber bis roter Farbtonnuance, die in der Natur weitverbreitet vorkommen und vielen Nahrungsmitteln eine charakteristische Färbung verleihen. Als wichtigste Vertreter dieser Stoffklasse seien β-Carotin, β-Apo-8'-carotinal, Canthaxanthin und Citranaxanthin genannt. Sowohl für die Lebensmittel- und Futtermittelindustrie als auch für die pharmazeutische Technologie stellen diese synthetisch herstellbaren Substanzen z.B. als Ersatz für künstliche Farbstoffe wichtige Farbkörper dar und sind z.B. wegen ihrer Pro-Vitamin-A-Aktivität von Interesse. Daneben häufen sich die Hinweise, daß z.B. β-Carotin als Prophylaktikum gegen Krebserkrankungen wirksam ist.

Alle Carotinoide sind in Wasser unlöslich, während in Fetten und Ölen eine ebenfalls nur geringe Löslichkeit gefunden wird. Diese begrenzte Löslichkeit sowie die hohe Oxidationsempfindlichkeit stehen einer direkten Anwendung der relativ grobkörnigen bei der Synthese erhaltenen Produkte in der Einfärbung von Lebens- und Futtermitteln entgegen, da die Substanzen in grobkristalliner Form nur schlecht resorbiert werden und somit nur schlechte Färbungsergebnisse liefern. Diese für die praktische Verwendung der Carotinoide nachteiligen Effekte wirken sich insbesondere im wäßrigen Medium aus, da sie darin gänzlich unlöslich sind.

Zur Verbesserung der Farbausbeuten und zur Erhöhung der Resorbierbarkeit bzw. Bioverfügbarkeit sind verschiedene Verfahren beschrieben worden, die alle das Ziel haben, die Kristallitgröße der Wirkstoffe zu verkleinern und auf einen Teilchengrößenbereich von kleiner 10 µm zu bringen.

Zahlreiche Methoden, u.a. beschrieben in Chimia 21, 329 (1967), WO 91/06292 sowie in WO 94/19411, bedienen sich dabei der Vermahlung von β-Carotin mittels einer Kolloidmühle und erzielen damit Partikelgrößen von 2 bis 10 µm.

Daneben existieren eine Reihe von kombinierten Emulgier-/Sprühtrocknungsverfahren, wie sie z.B. in DE-A-12 11 911 oder in EP-A-0 410 236 beschrieben sind.

Gemäß der europäischen Patentschrift EP-B-0 065 193 erfolgt die Herstellung von feinverteilten, pulverförmigen Carotinoidpräparaten dadurch, daß man ein Carotinoid in einem flüchtigen, mit Wasser mischbaren organischen Lösungsmittel bei Temperaturen zwischen 50°C und 200°C, gegebenenfalls unter erhöhtem Druck, innerhalb einer Zeit von weniger als 10 Sekunden löst. Aus der erhaltenen molekulardispersen Lösung wird das Carotinoid durch sofortiges schnelles Mischen mit einer wäßrigen Lösung eines Schutzkolloids bei Temperaturen zwischen 0°C und 50°C ausgefällt. Man erhält so ein kolloid-disperses β-Carotin-Hydrosol mit orangegelber Farbnuance. Anschließende Sprühtrocknung der Dispersion liefert ein freifließendes Trockenpulver, das sich in Wasser unter Bildung einer klaren, gelborange gefärbten Dispersion löst.

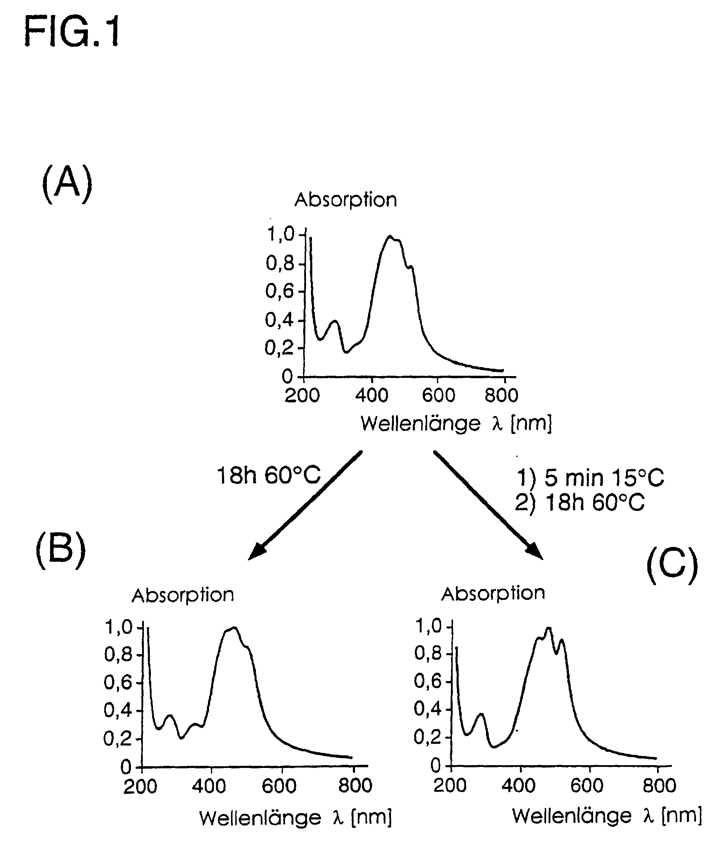

Wie FIG. 1A zeigt, erhält man nach der oben beschriebenen Vorgehensweise eine nanopartikuläre Wirkstoffdispersion, die sich durch das abgebildete Absorptionsspektrum und den daraus resultierenden gelb-orangenen, karottenfarbenen Farbton sowie durch einen Gehalt an all-trans Isomeren von typischerweise 76% auszeichnet.

Für die Anwendung der Carotinoide im wäßrigen Medium werden besonders hohe Anforderungen an die Konfektionierung gestellt. Eine möglichst feinverteilte Darreichung der Wirkstoffe ist dabei für die gewünschten Farbe- und Resorptionseigenschaften unbedingt erforderlich. Von großem Interesse sind Carotinoide mit einer breiten Variation an Färbeeigenschaften, verbunden mit einer guten Bioverfügbarkeit.

Es bestand daher die Aufgabe, ein Verfahren zur Herstellung von Carotinoiden in feinverteilter, pulverförmiger Form vorzuschlagen. Ferner sollten pulverförmige Carotinoid-Zubereitungen zur Verfügung gestellt werden, mit denen eine gezielte Farbwirkung eingestellt und zudem eine hohe Bioverfügbarkeit erzielt werden kann.

Diese Aufgabe wurde erfindungsgemäß gelöst mit einem Verfahren zur Herstellung von pulverförmigen, kaltwasserdispergierbaren Carotinoid-Zubereitungen, das dadurch gekennzeichnet ist, daß man
a) eine molekulardisperse Lösung eines Carotinoids gegebenenfalls zusammen mit einem Emulgator und/oder einem eßbaren Öl in einem flüchtigen, mit Wasser mischbaren, organischen Lösungsmittel bei höherer Temperatur herstellt und mit einer wäßrigen Lösung eines Schutzkolloids versetzt, wobei die hydrophile Lösungsmittelkomponente in die wäßrige Phase überführt wird und die hydrophobe Phase des Carotinoids als nanodisperse Phase entsteht,
b) das so erhaltene Hydrosol bei einer Temperatur zwischen 40°C bis 90°C für eine Zeitdauer von 5 bis 24 Stunden tempert und
c) das getemperte Hydrosol von dem Lösungsmittel und dem Wasser befreit und in ein wasserdispergierbares Trockenpulver überführt.

Die so erhältlichen Carotinoid-haltigen, kaltwasserdispergierbaren Trockenpulver lassen sich hervorragend zum Färben von Lebensund/oder Futtermitteln sowie als pharmazeutische Darreichungsform verwenden.

Schritt a) des erfindungsgemäßen Verfahrens kann im Prinzip wie in der EP-A-0 065 193 beschrieben durchgeführt werden, indem man das/die Carotinoid(e) gegebenenfalls zusammen mit einem Emulgator und/oder einem eßbaren Öl in einem flüchtigen, mit Wasser mischbaren organischen Lösungsmittel bei höherer Temperatur, z.B. zwischen ca. 50°C und 240°C, insbesondere 100°C bis 200°C, vorzugsweise 140°C bis 180°C, gegebenenfalls unter erhöhtem Druck löst.

Da die Einwirkung hoher Temperaturen den gewünschten hohen all-trans Isomerenanteil herabsetzen kann, löst man das/die Carotinoid(e) möglichst rasch, beispielsweise im Sekundenbereich, z.B. in 0,1 bis 10 Sekunden, besonders bevorzugt in weniger als 1 Sekunde. Zur raschen Herstellung der molekulardispersen Lösung kann die Anwendung von erhöhtem Druck, z.B. im Bereich von 20 bar bis 80 bar, vorzugsweise 30 bis 60 bar vorteilhaft sein.

Die so erhaltene molekulardisperse Lösung versetzt man unmittelbar anschließend mit der gegebenenfalls gekühlten wäßrigen Lösung eines Schutzkolloids in der Weise, daß sich eine Mischungstemperatur von etwa 35°C bis 80°C einstellt. Dabei wird die hydrophile Lösungsmittelkomponente in die wäßrige Phase überführt und die hydrophobe Phase des/der Carotinoid(e) entsteht als nanodisperse Phase.

Überraschenderweise wurde nun gefunden, daß sich, wie in FIG. 1B dargestellt, durch Tempern des Carotinoid-Hydrosols bei einer Temperatur von 40°C bis 90°C, vorzugsweise 50°C bis 70°C, die Absorption ins Kurzwellige verschiebt und man damit Farbtöne erzielen kann, die wesentlich gelber wirken als die der Ausgangsdispersion. Die cis-Isomerisierung schreitet dabei weiter fort, so daß z.B. nach 18- stündigem Tempern bei 60°C der Anteil der all-trans Konfiguration auf 60% gesunken ist. Man wird daher vorteilhafterweise den Tempervorgang zeitlich so beschränken, daß mindestens 50% an all-trans Konfiguration vorliegt. Die Dauer des Temperns liegt bei etwa 5 bis 24, insbesondere 12 bis 18 Stunden.

Es wurde weiterhin erfindungsgemäß festgestellt, daß man den trans-Isomerisierungsgrad deutlich erhöhen kann, wenn man, wie in FIG. 1C gezeigt, die Wirkstoffdispersion vor dem oben beschriebenen Tempern auf 0 bis 30°C, bevorzugt auf 10°C bis 20°C, insbesondere auf 15°C abkühlt. Dieses Abkühlen kann vorteilhafterweise mittels eines Wärmeaustauschers erfolgen. Von dessen Kapazität und der Durchflußmenge wird die Dauer des Abkühlens abhängig sein. In der Regel wird für das Abkühlen ein Zeitraum von etwa 1 Minute bis etwa 5 Minuten ausreichend sein, insbesondere bei kontinuierlicher Fahrweise. Erwärmt man die Dispersion anschließend auf z.B. etwa 60°C, so beobachtet man unerwarteterweise eine Rückisomerisierung, die sogar über den ursprünglichen Isomerisierungsgrad hinaus auf 81% all-trans Isomeren fortschreitet. Der Anteil an cis-Isomeren beträgt nur noch 19%. Damit verbunden ist eine deutliche Verschiebung des Absorptionspektrums in den langwelligen Bereich. Somit können Farbtöne erzielt werden, die wesentlich stärker orange wirken als die der Ausgangsdispersion.

Aufgrund des höheren all-trans Anteils des Carotinoid-Trockenpulvers ist eine höhere Bioverfügbarkeit [Jensen et al., Nutr. Rep. Int., 35, 413 (1987); Gaziano et al. Am. J. Clin. Nutr., 61, 1242 (1995)] und damit eine bessere Farbausbeute dieser Wirkstoffzubereitung gegeben.

Gegenstand der Erfindung sind daher auch pulverförmige, kaltwasserdispergierbare Carotinoid-Zubereitungen, erhältlich nach den eingangs beschriebenen Verfahren, dadurch gekennzeichnet, daß sie einen all-trans Isomerengehalt von mindestens 50% aufweisen.

Gegenstand der Erfindung sind außerdem pulverförmige, kaltwasserdispergierbare Carotinoid-Zubereitungen, erhältlich nach den eingangs beschriebenen Verfahren, dadurch gekennzeichnet, daß sie einen all-trans Isomerengehalt von mindestens 75% aufweisen.

Beide Varianten des erfindungsgemäßen Verfahrens führen zu unterschiedlichen morphologischen Eigenschaften der präzipitierten Wirkstoffpartikel. So sind die gefällten Wirkstoffpartikel nach Tempern bei 40°C bis 90°C, insbesondere 50°C bis 70°C im wesentlichen sphärisch bei einem Durchmesser von typischerweise 200 nm.

Gemäß der Verfahrensvariante, nach der man das Hydrosol vor dem Tempern für mindestens 1 Minute auf eine Temperatur von 0°C bis 30°C abkühlt, wandeln sich die sphärischen Teilchen in längliche, prolate Teilchen um ("Kaffeebohnenstruktur") und weisen dabei typischerweise eine Länge von 200 bis 300 nm und eine Dicke von 100 bis 150 nm auf.

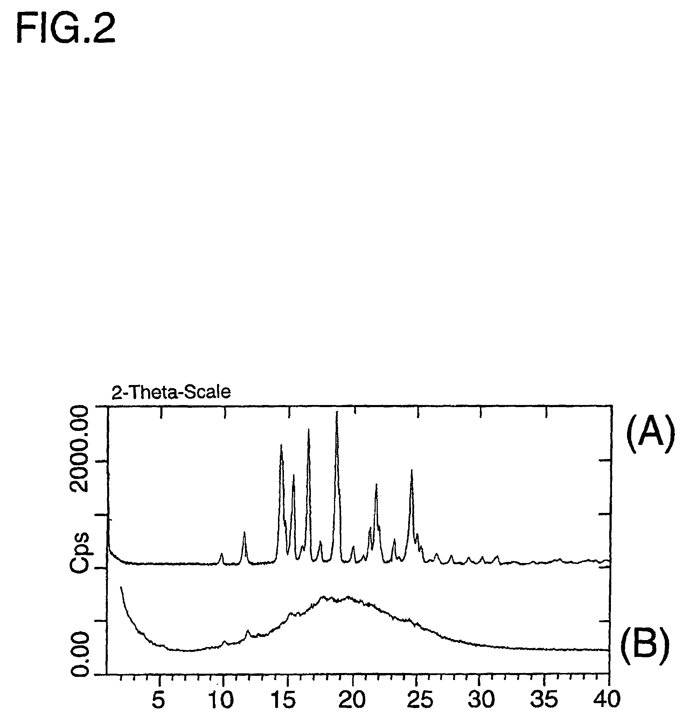

Die nach den erfindungsgemäßen Verfahrensvarianten hergestellten Trockenpulver zeigen im Röntgenbeugungsdiagramm überraschenderweise das in FIG. 2b dargestellte Phänomen. Am Beispiel einer 20 %igen β-Carotin-Formulierung ist zu erkennen, daß die Wirkstoffpartikel weitgehend röntgenamorph sind, d.h. der kristalline Anteil beträgt noch etwa 10%. Die beobachteten Röntgenreflexe weisen darüberhinaus auf eine Struktur hin, die mit der Kristallstruktur des kristallinen β-Carotins, dessen Röntgenbeugungsdiagramm in FIG. 2a abgebildet ist, nicht identisch ist. Analysen der Röntgenspektren der erfindungsgemäß hergestellten Wirkstoffpartikel zeigen, daß diesen Spektren eine Struktur mit mehr als zwei Molekülen in der Elementarzelle zugrunde liegen muß. Dagegen weist die bekannte Kristallstruktur des β-Carotins 2 Moleküle in der Elementarzelle auf.

Gegenstand der vorliegenden Erfindung sind auch pulverförmige, kaltwasserdispergierbare Carotinoid-Zubereitungen, erhältlich nach den eingangs beschriebenen Verfahren, dadurch gekennzeich net, daß sie einen röntgenamorphen Anteil zwischen 70 und 100% aufweisen und daß der verbleibende kristalline Anteil eine von der kristallinen Struktur des reinen Carotinoids abweichende Kristallmodifikation aufweist.

Eine hohe Amorphizität der Wirkstoffe im Trockenpulver läßt wiederum auf eine höhere Bioverfügbarkeit und Farbausbeute schließen.

Die Carotinoide, die bei der Durchführung der Erfindung eingesetzt werden können, sind die bekannten, zugänglichen, natürlichen oder synthetischen Vertreter dieser Klasse von Verbindungen, die als farbgebende Mittel brauchbar sind, z.B. β-Carotin, Lycopin, Bixin, Zeaxanthin, Cryptoxanthin, Citranaxanthin, Lutein, Canthaxanthin, Astaxanthin, β-Apo-4'-carotinal, β-Apo-8'-carotinal, β-Apo-12'-carotinal, β-Apo-8'-carotinsäure sowie Ester von hydroxy- und carboxyhaltigen Vertretern dieser Gruppe, z.B. die niederen Alkylester und vorzugsweise die Methyl- und Ethylester. Besonders bevorzugt werden die bisher technisch gut zugänglichen Vertreter wie β-Carotin, Canthaxanthin, Astaxanthin, β-Apo-8'-carotinal und β-Apo-8'-carotinsäureester verwendet.

Zur Durchführung des erfindungsgemäßen Verfahrens sind vor allem wassermischbare, thermisch stabile, flüchtige, nur Kohlenstoff, Wasserstoff und Sauerstoff enthaltene Lösungsmittel wie Alkohole, Ether, Ester, Ketone und Acetale geeignet. Vorzugsweise werden Ethanol, n-Propanol, Isopropanol, 1,2-Butandiol-1-methylether, 1,2-Propandiol-1-n-propylether oder Aceton verwendet. Allgemein verwendet man zweckmäßig solche Lösungsmittel, die mindestens zu 10% wassermischbar sind, einen Siedepunkt unter 200°C aufweisen und/oder weniger als 10 Kohlenstoffe haben.

Als Schutzkolloide werden beispielsweise Gelatine, Fischgelatine, Stärke, Dextrin, Pflanzenproteine, Pektin, Gummi-Arabikum, Kasein, Kaseinat oder Mischungen davon verwendet. Es können aber auch Polyvinylalkohol, Polyvinylpyrrolidon, Methylcellulose, Carboxymethylcellulose, Hydroxypropylcellulose und Alginate eingesetzt werden. Bezüglich näherer Einzelheiten wird auf R.A. Morton, Fat Soluble Vitamins, Intern. Encyclopedia of Food and Nutrition, Bd.9, Pergamon Press 1970, S. 128-131, verwiesen. Zur Erhöhung der mechanischen Stabilität des Endproduktes ist es zweckmäßig, dem Kolloid einen Weichmacher zuzusetzen, wie Zucker oder Zuckeralkohole, z.B. Saccharose, Glucose, Lactose, Invertzucker, Sorbit, Mannit oder Glycerin. vorzugsweise 10 bis 150 Gew.%, besonders bevorzugt 20 bis 80 Gew.%, bezogen auf das/die Carotinoid(e), verwendet werden.

Unter Umständen kann es auch vorteilhaf t sein, zusätzlich in der Lösungsmittel-Phase ein physiologisch zugelassenes Öl wie beispielsweise Sesamöl, Maiskeimöl, Baumwollsaatöl, Sojabohnenöl oder Erdnußöl sowie Ester mittelkettiger pflanzlicher Fettsäuren in einer Konzentration von 0 bis 500 Gew.%, vorzugsweise 10 bis 300 Gew.%, besonders bevorzugt 20 bis 100 Gew.%, bezogen auf das/die Carotinoid(e), zu lösen, das dann gemeinsam mit den Wirkstoffen und den genannten Zusatzstoffen beim Mischen mit der wässrigen Phase extrem feinteilig ausgefällt wird.

Je nach Art und Menge des verwendeten Schutzkolloids erhält man eine tiefgefärbte viskose Flüssigkeit, die im Falle eines gelierfähigen Kolloids gelartig erstarrt. Die Entfernung des Lösungsmittels (Schritt c) kann je nach Siedepunkt in an sich bekannter Weise, z.B. durch Destillation, gegebenenfalls unter vermindertem Druck, oder durch Extraktion mit einem mit Wasser nicht mischbaren Lösungsmittel, erfolgen. In diesem Fall hat es sich als zweckmäßig und möglich erwiesen, das bei Verwendung von Isopropanol erhaltene Azeotrop ohne Wasserentfernung unmittelbar als Lösungsmittel einzusetzen. Vorzugsweise erfolgt die Lösungsmittelabtrennung jedoch gemeinsam mit der Entfernung des Wassers durch Sprühtrocknung oder Sprühgranulation.

Man erhält ein Trockenpulver, das bei Verwendung eines wasserlöslichen Kolloids erneut in Wasser unter Erzielung einer gleichmäßigen Feinverteilung des Wirkstoffes im Korngrößenbereich kleiner 1 µm gelöst werden kann. Im photochemischen Stabilitätstest erweist sich das so erhaltene Wirkstoff-Hydrosol trotz der Feinverteilung als außerordentlich stabil.

Die erfindungsgemäßen Zubereitungen eignen sich aufgrund ihrer guten Kaltwasserdispergierbarkeit hervorragend als Lebensmittelfarbstoffe, speziell für Erfrischungsgetränke. Sie können auch anderen Lebensmitteln zugegeben werden, beispielsweise Backmischungen oder Puddingpulvern. Weiterhin eignen sich die Trockenpulver für die Herstellung von Präparaten zur Nahrungsergänzung mit Vitaminen im Human- und Tierbereich sowie für die Herstellung pharmazeutischer Präparate.

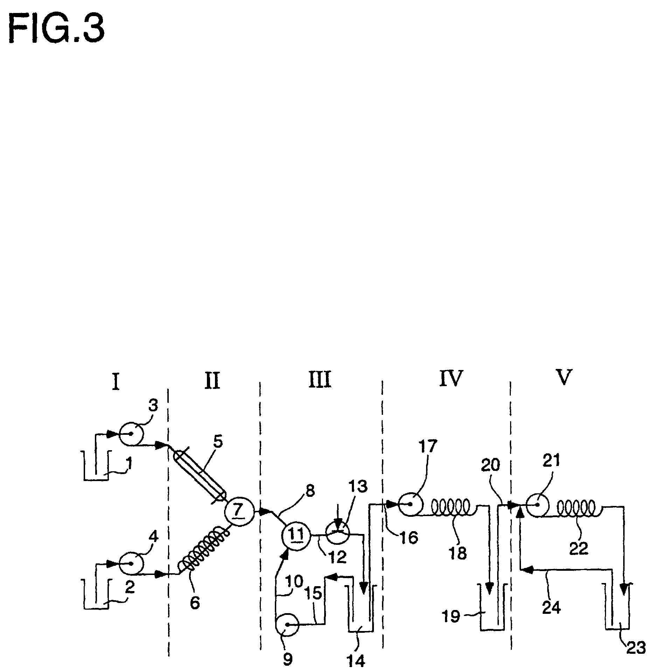

Im einzelnen kann man das erfindungsgemäße Verfahren beispielsweise mit einer Apparatur, wie sie in FIG. 3 schematisch dargestellt ist, wie folgt durchführen:

Die Apparatur gliedert sich in die Teile I bis V. Teil II ist der Hochtemperaturabschnitt, während in den übrigen Apparateteilen die Temperaturen weniger als 90°C betragen.

Im Gefäß (1) wird eine Suspension des/der Carotinoid(e) in dem ausgewählten Lösungsmittel in Konzentrationen von 2 bis 40 Gew.%, bezogen auf die Mischung, gegebenenfalls unter Zusatz von 0,1 bis 40 Gew.% an Stabilisatoren, vorgelegt. Gefäß (2) enthält das Lösungsmittel ohne Beimischung des/der Carotinoids(e). Über die Pumpen (3) bzw. (4) werden die Wirkstoff-Suspension und das Lösungsmittel der Mischkammer (7) zugeführt, wobei das Mischungsverhältnis durch Wahl der jeweiligen Förderleistung der Pumpen vorgegeben werden kann und so gewählt wird, daß je nach Lösungsmittel und verweilzeit eine Carotinoid-Konzentration in der Mischkammer von bis zu 10 Gew.% bezogen auf die Lösung, entsteht. Das Mischkammervolumen (7) ist so bemessen, daß bei der gewählten Förderleistung der Pumpen (3) und (4) die Verweilzeit in (7) vorzugsweise weniger als 1 Sekunde beträgt.

Das Lösungsmittel wird vor Eintritt in die Mischkammer über den Wärmeaustauscher (6) auf die gewünschte Temperatur gebracht, während die Wirkstoffsuspension durch Zuführung über die thermisch isolierte Zuleitung (5) bei Temperaturen unterhalb 80°C gehalten wird. Durch turbulente Mischung in (7) erfolgt im Temperaturbereich 50 bis 240°C, vor allem 100 bis 200°C, vorzugsweise jedoch bei 140 bis 180°C, die Lösung des Wirkstoffes und die erhaltene Lösung tritt über die Zuleitung (8) nach kurzer Verweilzeit, vorzugsweise von weniger als 1 Sekunde, in die zweite Mischkammer (11) ein, in der durch Zumischen von Wasser oder einer wäßrigen Schutzkolloid-Lösung über die Pumpe (9) und die Zuleitung (10) die Ausfällung des Wirkstoffes in kolloiddisperser Form erfolgt. Über Leitung (12) wird sodann die feinteilige Wirkstoffdispersion über das Überdruckventil (13) ausgetragen und dem Vorratsgefäß (14) zugeführt. Zur Erzielung einer möglichst hohen Wirkstoffkonzentration kann die Dispersion über die Saugleitung (15) im Kreis geführt werden.

Aus dem Vorratsgefäß (14) wird die Wirkstoffdispersion mit einer Temperatur zwischen 35 und 80°C, vorzugsweise zwischen 40 und 60°C über die Pumpe (17), Leitung (16) und den Wärmeaustauscher (18) in den Behälter (19) gefördert. Je nach der gewünschten Produktqualität wird die Temperatur der Wirkstoffdispersion im Wärmeaustauscher (18) entweder bei 35 bis 80°C, vorzugsweise bei 40 bis 60°C gehalten, oder für eine Zeit von mindestens 1 Minute, vorzugsweise 5 Minuten auf 0 bis 30°C, vorzugsweise ca. 15°C, abgesenkt. Vom Behälter (19) aus wird die Dispersion über die Zuleitung (20) und über die Pumpe (21) durch den Wärmeaustauscher (22) in das Gefäß (23) gefördert. Im Wärmeaustauscher (22) erfolgt eine Erwärmung auf insbesondere 50°C bis 70°C, vorzugsweise auf ca. 60°C. Zur Erzielung einer Temperzeit von 5 bis 24 Stunden, vorzugsweise 12 bis 18 Stunden, kann die Dispersion über die Saugleitung (24) im Kreis geführt werden.

Aus der Dispersion kann ein pulverförmiges Präparat in an sich bekannter Weise, z.B. gemäß den Angaben der DE-A-25 34 091 durch Sprühtrocknung oder durch Sprühkühlung oder durch Einhüllung der Teilchen, Abtrennen und Trocknen im wirbelbett erfolgen.

In den nachfolgenden Beispielen wird die Durchführung des erfindungsgemäßen Verfahrens näher erläutert.

### Beispiel 1:

63 g β-Carotin (grobkristalline Syntheseware) und 25 g Pflanzenöl wurden in einer Lösung von 11 g dl-α-Tocopherol und 21 g Ascorbylpalmitat in 250 g Isopropanol bei 25°C suspendiert und bei einer Mischungstemperatur von 175°C mit 560 g Isopropanol/Wasser (88/12) in der Mischkammer (7) (Fig. 3) bei einer Verweilzeit von 0,4 Sekunden gemischt. Die dabei entstandene molekulardisperse Lösung wurde unmittelbar anschließend über die Zuleitung (8) der Mischkammer (11) zugeführt, in der durch Vermischen mit 5600 g einer wäßrigen Gelatinelösung, die neben 170 g Gelatine, 285 g Zucker enthielt, das β-Carotin in kolloiddisperser Form bei einer Temperatur von 45°C ausfiel. Der gesamte Prozeß erfolgte unter Einstellung des Druckbegrenzungsventils (13) auf 30 bar, um eine Verdampfung des Lösungsmittels während der Feinverteilung zu vermeiden.

Die im Auffanggefäß (14) erhaltene kolloid-disperse β-Carotin-Dispersion wurde ohne vorherige Abkühlung im Wärmeaustauscher (22) auf 60°C erwärmt und 18 Stunden bei dieser Temperatur getempert, wodurch man eine kolloid-disperse β-Carotin-Dispersion mit gelber Farbtonnuance erhielt.

Durch Sprühtrocknung der Dispersion wurde ein freifließendes Trockenpulver erhalten, das sich in Wasser unter Bildung einer klaren, gelb gefärbten Dispersion löst.

### Beispiel 2:

Wie in Beispiel 1 beschrieben, wurde β-Carotin in kolloiddisperser Form ausgefällt. Die im Auffanggefäß (14) erhaltene β-Carotin-Dispersion wurde im Wärmeaustauscher (18) (Fig. 3) auf 15°C abgekühlt und 5 Minuten bei 15°C thermostatisiert. Anschließend erfolgte mittels Wärmeaustauscher (22) das 18-stündige Tempern der Dispersion bei 60°C, wodurch man eine kolloid-disperse β-Carotin-Dispersion mit orangener Farbtonnuance erhielt.

Durch Sprühtrocknung der Dispersion wurde ein freifließendes Trockenpulver erhalten, das sich in Wasser unter Bildung einer klaren, orange gefärbten Dispersion löst.

## Patentansprüche

1. Verfahren zur Herstellung von pulverförmigen, kaltwasserdispergierbaren Carotinoid-Zubereitungen **dadurch gekennzeichnet, daß** man
a) eine molekulardisperse Lösung eines Carotinoids gegebenenfalls zusammen mit einem Emulgator und/oder einem eßbaren Öl in einem flüchtigen, mit Wasser mischbaren, organischen Lösungsmittel bei höherer Temperatur herstellt und mit einer wäßrigen Lösung eines Schutzkolloids versetzt, wobei die hydrophile Lösungsmittelkomponente in die wäßrige Phase überführt wird und die hydrophobe Phase des Carotinoids als nanodisperse Phase entsteht,
b) das so erhaltene Hydrosol bei einer Temperatur zwischen 40°C bis 90°C **für eine Zeitdauer von 5 bis 24 Stunden** tempert und
c) das getemperte Hydrosol von dem Lösungsmittel und dem Wasser befreit und in ein wasserdispergierbares Trockenpulver überführt.

2. Verfahren nach Anspruch 1, **dadurch gekennzeichnet, daß** man die molekulardisperse Carotinoidlösung bei Temperaturen von 50°C bis 240°C herstellt und unmittelbar anschließend mit der wäßrigen Lösung des Schutzkolloids versetzt, wobei sich eine Mischungstemperatur von etwa 35°C bis 80°C einstellt.

3. Verfahren nach den Ansprüchen 1 und 2, **dadurch gekennzeichnet, daß** man das Hydrosol vor dem Tempern in Schritt b) **für eine Zeitdauer von 1 bis 5 Minuten** auf eine Temperatur von 0°C bis 30°C abkühlt.

4. Verfahren nach den Ansprüchen 1 bis 3, **dadurch gekennzeichnet, daß** man in Schritt a) eine molekulardisperse Lösung, enthaltend das/die Carotinoid(e) und einen Emulgator im Mengenverhältnis 0 bis 200 Gew.%, bezogen auf das/die Carotinoid(e) herstellt.

5. Verfahren nach den Ansprüchen 1 bis 4, **dadurch gekennzeichnet, daß** man in Schritt a) eine molekulardisperse Lösung, enthaltend das/die Carotinoid(e) und ein eßbares Öl im Mengenverhältnis 0 bis 500 Gew.%, bezogen auf das/die Carotinoid(e) herstellt.

6. Pulverförmige, kaltwasserdispergierbare Carotinoid-Zubereitungen, erhältlich nach einem Verfahren, definiert gemäß den Ansprüchen 1 bis 5, **dadurch gekennzeichnet, dass** sie ein eßbares Öl in einer Konzentration von 20 bis 100 Gew.-%, bezogen auf das Carotinoid enthalten.

7. Pulverförmige, kaltwasserdispergierbare Carotinoid-Zubereitungen nach Anspruch 6, **dadurch gekennzeichnet, daß** sie einen röntgenamorphen Anteil zwischen 70 und 100 % aufweisen und daß der verbleibende kristalline Anteil eine von der kristallinen Struktur des reinen Carotinoids abweichende Kristallmodifikation aufweist.

8. Pulverförmige, kaltwasserdispergierbare Carotinoid-Zubereitungen nach Anspruch 6, **dadurch gekennzeichnet, daß** sie einen all-trans Isomerengehalt von mindestens 50% aufweisen.

9. Verwendung der pulverförmigen, kaltwasserdispergierbaren Carotinoid-Zubereitungen, definiert gemäß den Ansprüchen 6 bis 8 als Zusatz zu Lebensmitteln, Pharmazeutika und/oder Tierfuttermitteln.

## Claims

1. A process for producing carotenoid preparations in the form of coldwater-dispersible powders, which comprises
a) preparing a molecular-disperse solution of a carotenoid, with or without an emulsifier and/or an edible oil, in a volatile, water-miscible, organic solvent at elevated temperature and adding therein an aqueous solution of a protective colloid, whereupon the hydrophilic solvent component is transferred into the aqueous phase, and the hydrophobic phase of the carotenoid results as nanodisperse phase,
b) heating the resulting hydrosol at from 40°C to 90°C for a period of from 5 to 24 hours and
c) removing the solvent and the water from the heated hydrosol, and converting it into a water-dispersible dry powder.

2. A process as claimed in claim 1, wherein the molecular-disperse carotenoid solution is prepared at from 50°C to 240°C and, immediately thereafter, the aqueous solution of the protective colloid is added, whereupon the temperature of the mixture adjusts itself to about 35°C to 80°C.

3. A process as claimed in claims 1 and 2, wherein the hydrosol is, before the heating in step b), cooled to from 0°C to 30°C for a period of from 1 to 5 minutes.

4. A process as claimed in any of claims 1 to 3, wherein a molecular-disperse solution comprising the carotenoid(s) and an emulsifier in an amount of from 0 to 200% of the weight of the carotenoid(s) is prepared in step a).

5. A process as claimed in any of claims 1 to 4, wherein a molecular-disperse solution comprising the carotenoid(s) and an edible oil in an amount of from 0 to 500% of the weight of the carotenoid(s) is prepared in step a).

6. A carotenoid preparation in the form of a coldwater-dispersible powder, obtainable by a process as defined in any of claims 1 to 5, which comprises an edible oil in a concentration of from 20 to 100% of the weight of the carotenoid.

7. A carotenoid preparation in the form of a coldwater-dispersible powder as claimed in claim 6, wherein the X-ray amorphous content is from 70 to 100%, and wherein the remaining crystalline content has a crystal modification differing from the crystal structure of the pure carotenoid.

8. A carotenoid preparation in the form of a coldwater-dispersible powder as claimed in claim 6, which has an all-trans isomer content of at least 50%.

9. The use of a carotenoid preparation in the form of a coldwater-dispersible powder as defined in any of claims 6 to 8 as additive to pharmaceuticals or human or animal foods.

## Revendications

1. Procédé pour la préparation de compositions de caroténoïdes pulvérulentes, dispersables à froid dans l'eau, **caractérisé par le fait que**
a) on prépare une solution en dispersion moléculaire d'un caroténoïde, éventuellement conjointement avec un émulsifiant et/ou une huile comestible dans un solvant organique miscible avec l'eau, à température élevée, et on ajoute une solution aqueuse d'un colloïde protecteur, tandis que le composant de solvant hydrophile est transféré dans la phase aqueuse et que la phase hydrophobe du caroténoïde consiste en une phase nanodispersée,
b) on soumet l'hydrosol ainsi formé à une trempe à une température entre 40°C et 90°C pendant une durée de 5 à 24 heures et
c) on débarrasse du solvant et de l'eau l'hydrosol résultant de la trempe, et on le transforme en une poudre sèche hydrodispersable.

2. Procédé selon la revendication 1, **caractérisé par le fait qu'**on prépare la solution de caroténoïdes en dispersion moléculaire à des températures de 50°C à 240°C et on y ajoute ensuite directement la solution aqueuse du colloïde protecteur, tandis qu'il s'établit une température de mélange d'environ 35°C à 80°C.

3. Procédé selon les revendications 1 et 2, **caractérisé par le fait qu'**on refroidit l'hydrosol avant la trempe dans l'étape b) pendant une durée de 1 à 5 minutes, à une température de 0°C à 30°C.

4. Procédé selon les revendications 1 à 3, **caractérisé par le fait qu'**on prépare dans l'étape a) une solution en dispersion moléculaire, contenant le/les caroténoïde(s) et un émulsifiant dans le rapport de quantités de 0 à 200 % en poids, par rapport au(x) caroténoïde(s).

5. Procédé selon les revendications 1 à 4, **caractérisé par le fait qu'**on prépare dans l'étape a) une solution en dispersion moléculaire, contenant le/les caroténoïde(s) et une huile comestible dans le rapport de quantités de 0 à 500 % en poids, par rapport au(x) caroténoïde(s).

6. Compositions de caroténoïdes pulvérulentes, dispersables à froid dans l'eau, pouvant être obtenues par un procédé défini selon les revendications 1 à 5, **caractérisées par le fait qu'**elles contiennent une huile comestible dans une concentration de 20 à 100 % en poids, par rapport au caroténoïde.

7. Compositions de caroténoïdes pulvérulentes, dispersables à froid dans l'eau selon la revendication 6, **caractérisées par le fait qu'**elles présentent une fraction amorphe aux rayons X comprise entre 70 et 100 %, et que la fraction cristallisée restante présente une modification de cristaux s'écartant de la structure cristalline du caroténoïde pur.

8. Compositions de caroténoïdes pulvérulentes, dispersables à froid dans l'eau selon la revendication 6, **caractérisées par le fait qu'**elles présentent une teneur en isomère tout-trans d'au moins 50 %.

9. Utilisation des compositions de caroténoïdes pulvérulentes, dispersables à froid dans l'eau, définies selon les revendications 6 à 8, comme additif pour des aliments, des produits pharmaceutiques et/ ou des aliments pour animaux.
